# EUROPÄISCHE PATENTSCHRIFT

(11) **EP 0 898 930 B1**
(45) Veröffentlichungstag und Bekanntmachung des Hinweises auf die Patenterteilung: **31.07.2002**
(21) Anmeldenummer: 98116131.8
(22) Anmeldetag: 26.08.1998
(51) Int. Cl.: A61B 3/08, A61B 3/113

(54) **Verfahren und Anordnung zur Untersuchung des beidäugigen Sehens**
Method and device for the examination of binocular vision
Procédé et dispositif pour l'examen de la vision binoculaire

(30) Priorität: 26.08.1997 DE 19737119
(43) Veröffentlichungstag der Anmeldung: 03.03.1999
(73) Patentinhaber: Löw, Bernhard, Dr. med., 66333 Völklingen (DE)
(72) Erfinder: Löw, Bernhard, Dr. med., 66333 Völklingen (DE)
(74) Vertreter: Hering, Hartmut, Dipl.-Ing.

(56) Entgegenhaltungen:
- DE-A- 4 041 332
- DE-A- 19 505 399
- US-A- 2 724 305
- US-A- 4 347 508

## Beschreibung

Die Erfindung betrifft ein Verfahren und eine Anordnung zur Untersuchung des beidäugigen (binokularen) Sehens.

Die Untersuchung des beidäugigen Sehens oder des Binokularsehens kann in die Untersuchung der Motorik und in die Untersuchung der Sensorik gegliedert werden. Bei der Untersuchung der Motorik sollen Stellungsfehler (Schielwinkel) und Bewegungsfehler (Störungen des Bewegungsablaufs, Einschränkungen des Blickfeldes) erkannt und vermessen werden. Bei der Untersuchung der Sensorik werden unter anderem die Verschmelzung der Bildeindrücke beider Augen (Fusion) sowie das räumliche Sehen (Stereosehen) geprüft.

Bei der Untersuchung von Bewegungsstörungen der Augen oder bei der Schieldiagnostik werden Fehlstellungen der Augen gemessen. Solche Fehlstellungen können verursacht sein durch Über- oder Unterfunktion eines oder mehrerer der sechs Augenmuskeln oder durch eine Störung eines oder mehrerer der drei Nerven, die die Muskelfunktionen steuern. Um Augenmuskelstörungen zu erfassen, führt man Schielwinkelmessungen in unterschiedlichen Blickrichtungen durch.

Zu einer möglichst exakten Beurteilung der Augenmuskelfunktion sind Prüfungen in möglichst vielen Blickrichtungen erforderlich. Mathematisch gesehen sind unendlich viele Blickrichtungen möglich. Physiologisch sinnvoll und für die Basisdiagnostik geeignet ist die Prüfung in den neun Hauptblickrichtungen. Diese sind: links oben, oben, rechts oben, links, geradeaus, rechts, links unten, unten, rechts unten. Die ermittelten Abweichungen der Blickrichtungen beider Augen kann man in Winkelgraden angeben oder in Prismendioptrien. Die Augenmuskelfunktionen lassen sich auch überprüfen mittels der sogenannten Koordimetrie. Hierbei werden Schielwinkel veranschaulicht, indem die Abweichungen der Blickrichtungen beider Augen von den Hauptblickrichtungen in einem Diagramm graphisch dargestellt werden. Geprüft wird mit sogenannten "Tangententafeln". Deren horizontal und vertikal gekrümmte Linien entstehen durch Projektion von Oberflächenkoordinaten einer Kugel auf eine im Abstand des Kugelradius befindliche Fläche. Der Kopf des Probanden befindet sich im Zentrum der Kugel und steht frontoparallel zur Projektionsfläche. Die sogenannten "Tangententafeln" sind immer nur für eine bestimmte Entfernung berechnet. Als Beispiele für "Tangententafeln" sind zu nennen der Hess-Schirm, der Lee-Screen, und der kürzlich vorgestellte Helmholtz-Schirm. Der Untersuchungsabstand beträgt jeweils 50 cm. Beim Hess-Schirm und beim Lee-Screen kann in einem Blickfeldbereich von etwa 70°, beim Helmholtz-Schirm in einem Blickfeldbereich von 100° geprüft werden. Durch das Zentrum des Schirms und durch die Schnittpunkte bestimmter horizontaler und vertikaler Linien ergeben sich Fixationspunkte für verschiedene Blickrichtungen. Die Bildtrennung kann auf unterschiedliche Art erfolgen. Am gebräuchlichsten ist die Bildtrennung durch komplementärfarbige Fixationsobjekte und komplementärfarbige Filtergläser. Der Untersucher richtet beispielsweise eine rote Lichtmarke auf einen Fixationspunkt. Der Proband, der eine Rot-Grün-Brille trägt, sieht die rote Lichtmarke nur mit dem Auge, vor dem sich das Rotglas befindet. Er soll die grüne Lichtmarke, die er nur mit dem anderen Auge sieht, mit der roten Lichtmarke zur Deckung bringen. Die Lage der grünen Markierung auf dem Schirm relativ zu den fixierten roten Punkten gibt die relative Lage der Augenachsen zueinander an. Sie wird zumindest in den 9 Hauptblickrichtungen geprüft, in ein Schema eingetragen und für die Diagnose ausgewertet.

Blickwinkel werden bestimmt durch die Kopfposition und die Lage der Gesichtslinie. Die Gesichtslinie ist die Gerade durch den Fixierpunkt und die Netzhautmitte (Foveola). Bei der Untersuchung von Bewegungsstörungen der Augen sind die Blickrichtungen des Patienten immer in Bezug zu seiner Kopfhaltung zu setzen. Es gibt hierzu bisher lediglich zwei prinzipielle Meßmöglichkeiten: Bei der ersten wird der Kopf des Patienten in einer Grundposition fixiert, und dann muß der Patient seine Blickrichtungen ändern. Bei der zweiten Möglichkeit muß der Patient immer in die Mitte beispielsweise einer Projektionswand blicken, wobei der gewünschte Blickwinkel, wie beispielsweise 30° nach oben, durch die gegensinnige Bewegung des Kopfes des Patienten 30° nach unten einzustellen ist. Bei der ersten Möglichkeit kommt beispielsweise ein Koordimeter nach Hess, Lee oder Helmholtz zum Einsatz, bei der zweiten beispielsweise die Tangentenskala nach Harms. Bei beiden Möglichkeiten gibt es also jeweils eine Konstante und eine Variable. Entweder der Kopf ist fixiert und die Blickrichtungen sind variabel, oder die Blickrichtung ist festgelegt und der Kopf wird bewegt. Die oben kurz beschriebenen Untersuchungsmethoden sind genauererläutert beispielsweise im Buch "Strabismus" von Herbert Kaufmann, 2. Aufl. Enke Verlag, 1995, S. 440 - 444, und in dem Buch "Augenärztliche Untersuchungsmethoden" von Straub, Kroll, Küchle, 2. Aufl. 1995, S. 628 ff und S. 639 ff.

Da bei der Untersuchung mittels einer Tangententafel Winkel gemessen werden, ist der erforderliche Untersuchungsabstand abhängig von der Größe der Tafel bzw. von der zur Verfügung stehenden Wandfläche. Weil Tangententafeln oder Koordimeterschirme nicht vergrößert oder verkleinert werden können, muß auf Einhalten des stets gleichen Untersuchungsabstands geachtet werden. Außerdem ist wichtig, daß die jeweilige Kopfposition genau eingehalten wird, damit Fehlmessungen vermieden werden.

Im Einsatz befindliche Koordimeter sind bereits bei einem Untersuchungsabstand von 50 cm relativ groß und schwer. Der Helmholtz-Schirm beispielsweise hat eine Breite und Höhe von 1,30 m. Würde man den Schirm kleiner machen, müßte der Patient näher an den Schirm heranrücken. Bei Sehobjekten, die in der Nähe liegen, muß akkommodiert werden und die Augen müssen sich gegenläufig bewegen, d.h. sie müssen konvergieren. Somit können sich die Einflüsse von Konvergenz und Akkommodation störend auf das Untersuchungsergebnis auswirken. Ideal wäre es, wenn man an einer sich im Unendlichen befindenden Tangententafel oder einem sich im Unendlichen befindenden Koordimeter prüfen könnte. Dann wäre keine Akkommodation erforderlich und die Sehachsen beider Augen lägen parallel zueinander.

Demgemäß ist der geringe Untersuchungsabstand ein wesentlicher Nachteil bei der Koordimetrie am Hess- oder Helmholtz-Schirm. Vergrößert man den Untersuchungsabstand, dann wird die benötigte Fläche für die Untersuchungseinrichtung zu groß. Bei einem Untersuchungsabstand von beispielsweise 2,5 m wären Akkommodation und Konvergenz zwar kaum noch störend, aber der Schirm müßte bereits eine Breite und eine Höhe von mehr als 5 m haben, wenn die Möglichkeit gegeben sein sollte, Schielwinkel bei Blickwendungen bis zu 45° graphisch darzustellen oder zu messen. Demgemäß ist auch der Platzbedarf für eine durch Akkommodation oder Konvergenz wenig gestörte Untersuchung bei den bisherigen Methoden nachteilig.

Die DE 40 41 332 C2 zeigt ein Beispiel für die Projektions-Koordimetrie. Dasselbe Prinzip ist in der US 5 302 981 gezeigt, wobei allerdings der Projektionsschirm längs einer Ausdehnungsrichtung zu einem Balken geschrumpft ist, der wahlweise waagrecht oder senkrecht positioniert wird.

Die WO 96 13 195 A1 betrifft Untersuchungen des beidäugigen Sehens unter Anwendung der Methode nach Harms bzw. der Methode nach Hess/-Helmholtz.

In Patent Abstracts of Japan zur JP 08-038 426 A ist ein "Joystick" dargestellt, mit dessen Hilfe ein Proband zwei Marken auf einem Bildschirm zur Deckung bringt, wonach ein Rechner den Versatz auswertet.

Gemäß der DE 195 02 337 A1 und der DE 195 05 399 A1 ist ein "gleitendes" Gesichtsfeld in Verbindung mit perimetrischen Untersuchungen realisiert, wobei das Koordinatenzentrum der momentanen Augenposition nachgeführt wird.

Die US 2 724 305 zeigt, wie sich die Kopfhaltung auf optischem Weg mittels eines am Kopf des Probanden befestigten Spiegels erfassen läßt. Aus der Kopfhaltung soll dann auf die Blickrichtung geschlossen werden.

Der Erfindung liegt die Aufgabe zugrunde, ein Verfahren zur Untersuchung motorischer und sensorischer Funktionen der Augen zu schaffen, bei dem der Untersuchungsabstand variierbar ist und bei dem zugleich auf eine Fixation des Kopfes verzichtet werden kann.

Diese Aufgabe wird gemäß dem Verfahren nach Patentanspruch 1 und gemäß der Anordnung nach Patentanspruch 8 gelöst. Weitere Merkmale der Erfindung ergeben sich aus den Unteransprüchen.

Insbesondere können gemäß der vorliegenden Erfindung in einem bestimmten Ausschnitt des Blickfeldes motorische und sensorische Funktionen überprüft werden, wobei eine Auswahl des jeweiligen Ausschnitts auf der Basis einer erfaßten Kopfposition des Patienten erfolgt.

Wenn bei der Untersuchung an herkömmlichen Tangententafeln oder an herkömmlichen Koordimeterschirmen die störenden Einflüsse von Konvergenz und Akkommodation gering sein sollen, dann müssen diese Vorrichtungen sehr groß sein. Das beidäugige Blickfeld hat in der Horizontalen eine Ausdehnung von 100°, in der Vertikalen eine Ausdehnung von etwa 90°. Nun sind aber die zu messenden Schielwinkel in der Regel viel kleiner, so daß in der Regel auch nur ein Teilbereich des gesamten Blickfelds für die Messung relevant ist. Will man beispielsweise eine Augenmuskelfunktion in der Blickrichtung 20° nach unten messen, dann wird der obere Bereich des Blickfeldes auf dem Schirm nicht gebraucht. Die für den jeweiligen Untersuchungsabschnitt nicht relevanten Bereiche des Schirms, der Tangententafel, der Fläche bzw. des Raums, auf die sich das Blickfeld, bzw. in den sich das Blickfeld projiziert, können somit auch außer acht gelassen werden. Dies erfolgt gemäß der Erfindung, indem aus dem realen Schirm, bzw. aus der realen Tangententafel mit unveränderlicher Größe ein virtueller Schirm, bzw. eine virtuelle Tangententafel variabler Größe gemacht wird. Die Daten der Blickfeldkoordinaten werden vorab im Rechner digital abgespeichert. Auf ihrer Basis werden die vom Patienten angezeigten Abweichungen berechnet. In welchem Blickfeldbereich gerade untersucht wird und welcher Ausschnitt des Koordinatensystems gegebenfalls mit dargestellt wird, hängt primär von der Position des Kopfes des Patienten ab.

Insbesondere werden also jeweilige Positionsdaten für eine Kopfposition eines Probanden im Raum erfaßt. Mit Hilfe dieser Positionsdaten und mit Hilfe der vorab im Rechner gespeicherten Blickfeldkoordinaten wird der jeweilige Prüfbereich des Blickfeldes entsprechend der Kopfposition in Echtzeit verschoben. Über im Rechner gespeicherte Programme können im jeweils ausschnittartig dargestellten Blickfeld unterschiedliche Sehobjekte zur Darstellung gebracht werden. Zur Prüfung der Motorik lassen sich beispielsweise die Fixationspunkte der Tangententafeln oder der Koordimeter anzeigen. Es können beliebige andere Sehobjekte dargestellt werden zur Prüfung sensorischer Funktionen wie beispielsweise der Bestimmung des Fusionsblickfeldes oder der Bestimmung der Fusionsbreite in unterschiedlichen Blickrichtungen, zur Erkennung prismatischer Nebenwirkungen von Brillengläsern sowie zur Prüfung der Sehschärfe in Abhängigkeit vom Durchblickpunkt des Brillenglases. Wesentlich bei diesem Verfahren ist somit, daß durch die jeweils ausschnittartige Darstellung des Blickfeldes zusammen mit den darin befindlichen Sehobjekten (Prüfobjekten, Fixationspunkten, Sehzeichen), die einer jeweiligen Kopfbewegung nachgeführt werden, der Blickwinkel stets konstant gehalten werden kann. Zugleich kann im gesamten Blickfeldbereich untersucht bzw. gemessen werden. Die bei den bisherigen Methoden durch falsche Kopfposition oder durch Änderung der Kopfposition während der Untersuchung möglichen Messfehler sind ausgeschlossen. Außerdem wird durch die ausschnittartige Darstellung des Blickfeldes der Platzbedarf verringert und/oder der Untersuchungsabstand vergrößert.

Wenn eine sensorische Funktion unter einem bestimmten Blickwinkel geprüft werden soll, wird der Proband entweder von einer Hilfsperson oder durch in die Prüffläche (Bildschirm, Projektionswand) eingeblendete Hinweise aufgefordert, seinen Kopf so lange zu bewegen, bis die Sehobjekte in der Prüffläche auftauchen. Wenn der Proband dann diese fixiert, ist der Blickwinkel definiert durch die Lage dieser Sehobjekte und die Stellung des Kopfes. Auf diese Weise können sehr leicht Kopfzwangshaltungen und die beispielsweise bei der Anpassung von Brillengläsern wichtige bevorzugte Kopfhaltung ermittelt werden. Wenn beispielsweise motorische Störungen in den 9 Hauptblickrichtungen untersucht werden sollen, muss der Kopf so lange bewegt bzw. in so viele Positionen gebracht werden, bis alle 9 Fixationsmarken aufgetaucht sind. Um das Auffinden der Fixationsmarken zu erleichtern, können Richtungshinweise beispielsweise in Form von Richtungspfeilen für die jeweils vorzunehmende Änderung der Kopfhaltung eingeblendet werden. Hält der Patient seinen Kopf gerade (frontoparallel zur Projektionseinrichtung), dann wird die der Blickrichtung "geradeaus" entsprechende Fixationsmarke in der Mitte der Projektionsfläche dargestellt. Hält er seinen Kopf beispielsweise 15° nach rechts gewendet, dann erscheint in der Mitte der Projektionsfläche die Fixationsmarke, die bei gerade gehaltenem Kopf mit einer Blickwendung von 15° nach links fixiert werden könnte. Bewegt sich der Kopf während einer Untersuchung gewollt oder ungewollt, dann bewegen sich die Fixationsmarken innerhalb der Grenzen der Projektionsflächen mit. Dadurch bleibt der Blickwinkel konstant obwohl sich die Blickrichtung ändert. Die Darstellung der zu fixierenden und zu markierenden Punkte und das zu deren Positionsbestimmung erforderliche Bezugskoordinatensystem, das wahlweise ausschnittsartig mit dargestellt werden kann, erfolgt dynamisch, das heißt durch kontinuierliche Neuberechnung in Echtzeit auf der Basis der Ausgangskoordinaten.

Die beiden eingangs angegebenen Prinzipien der Schielwinkelbestimmung werden gemäß der Erfindung kombiniert, so daß es eine konstante, feste Kopfposition und eine festgelegte Blickrichtung des fixierenden Auges nicht mehr gibt, sondern statt dessen zwei Variable, wobei die Variabilität der Blickrichtung abhängig ist von der Größe des Bildschirms bzw. der Projektionseinrichtung. Das zu prüfende Blickfeld ist nicht mehr geräte- oder vorrichtungsbedingt eingeschränkt, weil der Kopf in allen Richtungen frei beweglich ist. Der Platzbedarf für die Untersuchungseinrichtung läßt sich wesentlich verringern und es wird ein größerer Untersuchungsabstand ermöglicht. Die Erfindung hat insbesondere den Vorteil, daß sich sensorische Prüfungen, beispielsweise bei Brillenunverträglichkeit, und motorische Störungen, beispielsweise bei Lähmung eines Augenmuskels, automatisch und selbstregistrierend durchführen lassen. Ein Proband, der zur Bildtrennung eine Rot-Grün-Brille trägt, wie es oben beschrieben ist, kann durch Bewegen seines Kopfes die Blickfeldbereiche so lange in vertikaler oder horizontaler Richtung verschieben, bis ein Fixationsobjekt unter einem bestimmten Blickwinkel erscheint. Das Fixationsobjekt kann beispielsweise ein roter bzw. grüner Ring sein, in dem ein komplementärfarbiges Kreuz zu zentrieren ist. Der Proband lenkt das Kreuz mit einer Markierungseinrichtung, einem "Joystick" oder einer "Maus", und bestätigt die Überlagerung beider Markierungen mittels Knopfdruck. Somit ergibt sich gemäß der Erfindung eine dynamische, über "Biofeedback" selbstregelnde Kopfeinstellung. Daraus ergibt sich die Möglichkeit für eine automatisch ablaufende Untersuchung. Diese kann der Proband selbständig und in sehr kurzer Zeit durchführen. Man kann die Untersuchung beispielsweise als Computerspiel konzipieren, bei dem der Proband lediglich die Aufgabe hat, Luftballons "abzuschießen", die er nacheinander durch Änderung der Kopfposition auf der Projektionsfläche zur Darstellung bringt. Die Anzahl der "abzuschießenden" Luftballons ergibt sich aus der Zahl der zu prüfenden Blickrichtungen.

Die Erfassung der Kopfposition kann am besten auf optoelektronischem Wege erfolgen. Dazu werden die Lichtreflexe von am Kopf des Probanden angebrachten Reflektoren über zumindest eine Videokamera erfasst und im Rechner ausgewertet. Es lassen sich auch Lichtquellen, vorzugsweise Laser, am Kopf des Probanden fixieren. Vorzugsweise werden drei Lichtquellen eingesetzt. Diese drei Lichtquellen lenken jeweils divergierende Lichtstrahlen auf eine Fläche, die wiederum mittels einer Videokamera abgetastet wird. Die Lichtstrahlen markieren auf der Fläche die Eckpunkte einer Pyramidenbasis. Aus der Position der Lichtpunkte läßt sich - bei bekanntem und gleichbleibendem Abstand zwischen Lichtquellen und Augen die Position der Augen im Raum berechnen. Wenn sich die Höhenlage des Kopfes während der Untersuchung nicht ändert, könnte eine Beschränkung auf zwei Lichtquellen vorgenommen werden. Anstelle einer optoelektronischen Erfassung der Kopfposition sind prinzipiell alle anderen Systeme zur Erfassung der Raumkoordinaten eines Objekts denkbar wie beispielsweise Neigungssensor-Systeme oder Ultraschall-Systeme.

Das erfindungsgemäße Verfahren und die zugehörige Anordnung sind flexibler einsetzbar, als dies bei herkömmlichen Untersuchungen des beidäugigen Sehens der Fall ist. Der Untersuchungsabstand ist frei veränderbar. Ein Fixieren des Kopfes eines Probanden ist nicht mehr erforderlich. Die Untersuchung kann programmgesteuert selbsttätig ablaufen, wodurch Zeit und Personal eingespart werden.

Zusammenfassend wird ein Verfahren zur Untersuchung des beidäugigen Sehens angegeben, das es ermöglicht, bei variablem Untersuchungsabstand mit einer dynamischen, über "Biofeedback" sich selbst regelnden Kopfeinstellung motorische und sensorische Störungen zu erfassen. Hierzu werden die Positionsdaten des Kopfes in Echtzeit numerisch erfaßt. Sie bestimmen unter Einbeziehung des Untersuchungsabstands den für den jeweiligen Untersuchungsabschnitt erforderlichen Blickfeldbereich. Die ermittelten Abweichungen werden gemessen durch kontinuierliche Neuberechnung in Abhängigkeit von der Kopfposition auf der Grundlage der digital gespeicherten Ausgangskoordinaten des Gesamtblickfeldes.

Eine beispielsweise Ausführungsform zum Durchführen des Verfahrens zur Untersuchung des beidäugigen Sehens gemäß der Erfindung wird nachfolgend anhand der Zeichnung im einzelnen erläutert, wobei die Zeichnung folgendes zeigt:
- Fig. 1: eine Anordnung zum Durchführen des Verfahrens zur Untersuchung des beidäugigen Sehens gemäß der Erfindung; und
- Fig. 2a und 2b: ein Beispiel für eine Bewegung des Kopfes eines Patienten, bei dem das beidäugige Sehen untersucht wird.

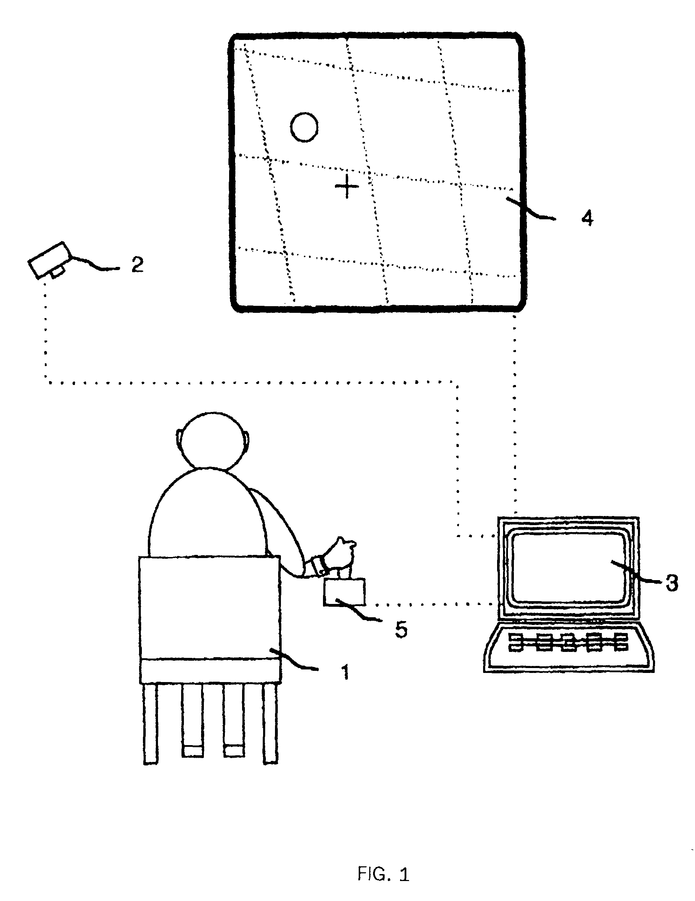

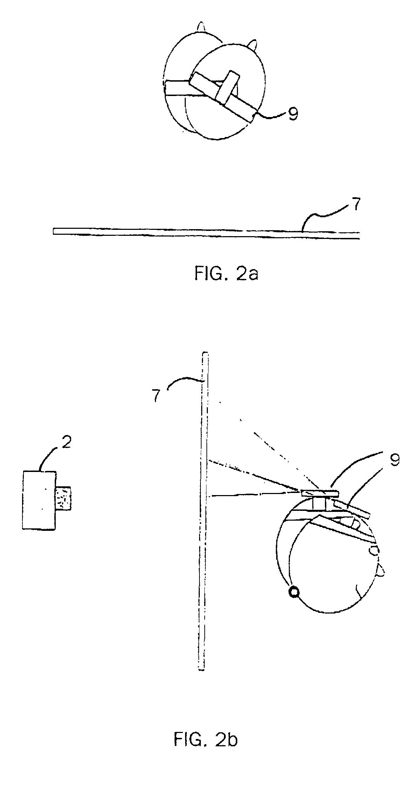

Fig. 1 zeigt eine Anordnung zum Durchführen des Verfahrens zur Untersuchung des beidäugigen Sehens gemäß der Erfindung. Dabei zeigt Fig. 1 einen Stuhl 1 für einen Patienten mit einer Erfassungseinrichtung 2 zum Ermitteln seiner Kopfposition, einen Computer 3 mit einem Speicher zum Speichern der Koordinatensysteme und einer Wähleinrichtung, einer Anzeigevorrichtung 4 und einer Markierungseinrichtung 5, wie beispielsweise einem sogenannten Joystick oder einer sogenannten Maus, mit welcher der Patient eine Untersuchungsstelle markieren kann, und eine Darstellung auf der Anzeigevorrichtung 4 mit einem Ausschnitt eines Koordinatensystems. Fig. 2 zeigt den Kopf eines Patienten, an welchem Richtstrahler 9 fixiert sind, in bezug zur Auftreffläche 7.

Gemäß den Fig. 2a und 2b werden Richtstrahler 9 am Kopf des Patienten derart fixiert, daß sie in bezug zu seinen Augen eine bestimmte und feste Position haben. Bei der Untersuchung geben die am Kopf des Patienten fixierten Richtstrahler 9 Licht an die Auftreffläche 7 ab. Die Auftreffläche 7 wird mittels der Erfassungseinrichtung 2 zur Ermittlung der Kopfposition des Patienten oder Probanden beobachtet. Die Erfassungseinrichtung 2 ist in dem Fall, in welchem die Richtstrahler 9 Lichtstrahler sind, beispielsweise eine Videokamera. Die Richtstrahler 9 können aber auch Ultraschallstrahler sein.

Vor der Untersuchung kann ein Untersuchungsbereich eingestellt werden. Wenn der ausgewählte Ausschnitt des Koordinatensystems angezeigt wird, muß der Patient die Markierungseinrichtung 5 zum Markieren der Untersuchungsstelle betätigen.

Auf einem Stuhl 1 sitzt der Proband. Nachdem die Erfassungseinrichtung 2 die Kopfposition erfaßt hat, wird das Meßergebnis in Form elektrischer Signale zum Computer 3 geführt, in dem diese binär codiert werden. Dann wird auf der Basis der codierten Signale ein Ausschnitt aus dem gewünschten Blickfeld ausgewählt, dessen Koordinaten im Speicher des Computers digital gespeichert sind. Zusammen mit den Blickfeldkoordinaten sind im Computer auch die Positionen der Fixationsobjekte, der Prüfzeichen und der Sehzeichen gespeichert. Die Auswahl kann beispielsweise mittels einer Tabelle erfolgen, die eine Zuordnung zwischen erfaßten Positionsdaten des Kopfes und den digital gespeicherten Werten für die Lage der Sehobjekte innerhalb des Blickfeldkoordinatensystems festlegt. Der Proband beobachtet die auf der Anzeigevorrichtung 4 dargestellten Objekte. Auf der Anzeigevorrichtung 4 sind die auf die Anzeigefläche projizierten Kugelkoordinaten des Blickfelds mit dargestellt. Mit einer Markierungseinrichtung 5, wie beispielsweise einer sogenannten Maus oder einem sogenannten Joystick, verschiebt der Proband das Kreuz, das er nur mit einem Auge sehen kann, so daß es sich mit dem Kreis, den er nur mit dem anderen Auge sehen kann, überlagert. Diese Position markiert er beispielsweise durch Betätigen einer Taste an der Markierungseinrichtung 5. Weil die Fusion der Bilder beider Augen aufgehoben ist, zeigen sich bei Vorliegen einer Störung Abweichungen, die vom Computer 3 gespeichert und ausgewertet werden können. Bewegt der Proband seinen Kopf, dann bewegen sich auch die auf der Projektionseinrichtung dargestellten Fixationspunkte, weil jeder Kopfposition ein bestimmter Ausschnitt des Blickfeldes zugeordnet ist.

Eine seitliche Bewegung des Kopfes ist in Fig. 2a gezeigt, und eine Bewegung des Kopfes nach oben oder nach unten ist in Fig. 2b gezeigt. In den Fig. 2a und 2b ist der Strahlengang der von der Quelle 9 ausgesendeten Strahlen für jeweils zwei Position des Kopfes zu sehen. Diese Strahlen werden dann mittels der Erfassungseinrichtung 2 erfaßt.

## Patentansprüche

1. Verfahren zur Untersuchung des beidäugigen Sehens, wobei bei unterschiedlichen Kopfpositionen und unterschiedlichen Blickrichtung eines Probanden motorische und sensorische Störungen erfaßt werden können, ohne daß die Notwendigkeit einer Kopffixierung besteht, indem Objekte als Fixationsmarken auf einem Bildschirm oder einer Projektionsfläche dargestellt werden und diese ihre Position in Abhängigkeit von Bewegungen des Kopfes ändern, wobei Bezugskoordinaten für die Lage der Fixationsobjekte im Blickfeld digital gespeichert sind und das Untersuchungsverfahren folgende Schritte aufweist:
a) Erfassen von Positionsdaten für eine Kopfposition des Probanden im Raum;
b) selbsttätiges Auswählen eines Ausschnitts des der jeweiligen Kopfposition zugeordneten Blickfeldbereichs; und
c) zur Darstellung Bringen des ausgewählten Ausschnitts des Blickfeldbereichs mit den gewünschten Fixationsmarken und Prüfzeichen und wahlweise mit dem zugrundeliegenden Koordinatensystem auf dem Bildschirm oder der Projektionsfläche.

2. Verfahren nach Anspruch 1, **dadurch gekennzeichnet, daß** ein Auswählen des Ausschnitts der gespeicherten Blickfeldbereiche des Bezugs-Koordinatensystems und der darin festgelegten Fixationspunkte dynamisch, über optomotorische Regelkreise des Probanden, d.h. über sognanntes Biofeedback, durch Bewegen des Kopfes erfolgen kann.

3. Verfahren nach Anspruch 2, **dadurch gekennzeichnet, daß** der auf dem Bildschirm oder der Projektionsfläche dargestellte Blickfeldbereich durch Bewegen des Kopfes solange weiterbewegt wird, bis ein Fixationspunkt erscheint, an dem eine Untersuchung stattfinden soll.

4. Verfahren nach Anspruch 3, **gekennzeichnet durch** Markieren des bereits mit einer Markierung versehenen Fixationspunkts für die Untersuchung **durch** den Probanden mittels einer Markierungseinrichtung (5).

5. Verfahren nach einem der vorangehenden Ansprüche, **gekennzeichnet durch** binäres Codieren der erfaßten Positionsdaten vor dem Auswählen des Ausschnitts der gespeicherten Blickfeldkoordinaten.

6. Verfahren nach einem der vorangehenden Ansprüche, **gekennzeichnet durch** Auswählen des Ausschnitts der gespeicherten Blickfeldkoordinaten auf der Basis der erfaßten Kopfpositionsdaten mittels einer Tabelle, die eine Zuordnung zwischen erfaßten Positionsdaten und den digital gespeicherten Werten der Blickfeldkoordinaten festlegt.

7. Verfahren nach einem der vorangehenden Ansprüche, **gekennzeichnet durch** Projizieren des ausgewählten Ausschnitts von Blickfeldbereichen auf eine Projektionsfläche.

8. Anordnung zum Durchführen des Verfahrens nach einem der vorangehenden Ansprüche, die folgendes aufweist:
a) einen Speicher zum digitalen Speichern des zur Schielwinkelmessung erforderlichen Bezugs-Koordinatensystems;
b) eine Erfassungseinrichtung (2) zum Erfassen von Positionsdaten für eine Kopfposition eines Probanden im Raum;
c) eine Wähleinrichtung zum selbsttätigen Auswählen eines Ausschnitts des gespeicherten Koordinatensystems in Abhängigkeit von den erfaßten Positionsdaten für die Kopfposition des Probanden; und
d) eine Anzeigevorrichtung (4), die den ausgewählten Ausschnitt des gespeicherten Koordinatensystems zur Darstellung bringt,
wobei der Speicher und die Wähleinrichtung in einem Computer (3) vorgesehen sind, dem die Positionsdaten von der Erfassungsvorrichtung (2) zugeführt werden.

9. Anordnung nach Anspruch 8, **dadurch gekennzeichnet, daß** die Anzeigevorrichtung (4) ein Bildschirm oder eine Projektionseinrichtung ist.

10. Anordnung nach Anspruch 8 oder 9, **dadurch gekennzeichnet, daß** weiterhin eine Markierungseinrichtung (5) am Computer (3) vorgesehen ist, mit welcher der Proband eine bereits markierte Untersuchungsstelle im Koordinatensystem markiert.

11. Anordnung nach einem der Ansprüche 8 bis 10, **dadurch gekennzeichnet, daß** die Erfassungseinrichtung (2) von einer am Kopf des Probanden mit festem Abstand zu seinen Augen angeordneten Quelle (9) ausgesendete Richtstrahlen erfaßt und zum Computer (3) weiterleitet.

12. Anordnung nach Anspruch 11, **dadurch gekennzeichnet, daß** die Richtstrahlen Lichtstrahlen, vorzugsweise Laserstrahlen, sind.

13. Anordnung nach einem der Ansprüche 11 oder 12, **dadurch gekennzeichnet, daß** die Erfassungseinrichtung (2) eine Videokamera (8) aufweist, die die Positionsdaten durch Abtasten von Auftreffpunkten der auf die als Mattglasscheibe ausgebildeten Auftreffläche (7) gerichteten Richtstrahlen erfaßt.

14. Anordnung nach einem der Ansprüche 8 bis 11, **dadurch gekennzeichnet, daß** die Erfassungseinrichtung (2) aus einem Ultraschall-System oder einem Neigungssensor-System gebildet ist.

## Claims

1. Process for the examination of binocular vision, wherein motor and sensory disturbances may be recorded at different head positions and different viewing direction of a test person without there being the necessity for fixing the head, by representing objects as fixation marks on a screen or a projection surface and they change their position as a function of movements of the head, wherein reference coordinates for the position of the fixation objects in the viewing field are stored digitally and the examination process has the following steps:
a) recording positional data for the head position of the test person in the room;
b) automatic selection of a cutout of the viewing field range assigned to the particular head position; and
c) to show the selected cutout of the viewing field range with the required fixation marks and test characters and if required with the underlying coordinate system on the screen or the projection surface.

2. Process according to claim 1, **characterised in that** selection of the cutout of the stored viewing field ranges of the reference coordinate system and the fixation points defined therein may take place dynamically via optomotor control circuits of the test person, that is via so-called biofeedback, by moving the head.

3. Process according to claim 2, **characterised in that** the viewing field range shown on the screen or the projection surface continues to be moved by moving the head until a fixation point appears, at which an examination should take place.

4. Process according to claim 3, **characterised by** marking the fixation point for the examination already provided with a marking by the test person by means of a marking device (5).

5. Process according to one of the preceding claims, **characterised by** binary coding of the recorded positional data before selecting the cutout of the stored viewing field coordinates.

6. Process according to one of the preceding claims, **characterised by** selecting the cutout of the stored viewing field coordinates on the basis of the recorded head positional data by means of a chart, which determines an assignment between recorded positional data and the digitally stored values of the viewing field coordinates.

7. Process according to one of the preceding claims, **characterised by** projecting the selected cutout of viewing field ranges on a projection surface.

8. Arrangement for carrying out the process according to one of the preceding claims, which has the following:
a) a memory for digital storage of the reference coordinate system required for strabometry;
b) a recording device (2) for recording positional data for a head position of a test person in the room;
c) a selecting device for automatic selection of a cutout of the stored coordinate system as a function of the recorded positional data for the head position of the test person; and
d) a display device (4), which shows the selected cutout of the stored coordinate system,
wherein the memory and the selection device are provided in a computer (3), to which the positional data are supplied from the recording device (2).

9. Arrangement according to claim 8, **characterised in that** the display device (4) is a screen or a projection device.

10. Arrangement according to claim 8 or 9, **characterised in that** a marking device (5) is also provided on the computer (3), with which the test person marks an already marked examination point in the coordinate system.

11. Arrangement according to one of claims 8 to 10, **characterised in that** the recording device (2) records directional beams emitted from a source (9) arranged on the head of the test person at a fixed distance from his eyes and conveys them to the computer (3).

12. Arrangement according to claim 11, **characterised in that** the directional beams are light beams, preferably laser beams.

13. Arrangement according to one of claims 11 or 12, **characterised in that** the recording device (2) has a video camera (8), which records the positional data by scanning impinging points of the directional beams directed onto the impinging surface (7) formed as a mat glass sheet.

14. Arrangement according to one of claims 8 to 11, **characterised in that** the recording device (2) is formed from an ultrasound system or an inclination sensor system.

## Revendications

1. Procédé d'examen de la vue binoculaire permettant de saisir des troubles moteurs et sensoriels pouvant être saisis à différentes positions de la tête et à différentes directions du regard sans qu'il soit nécessaire de fixer la tête, en représentant des objets servant de marques de fixation sur un écran ou sur une surface de projection, objets qui changent leur position en fonction des mouvements de la tête, les coordonnées de référence pour la position des objets de fixation dans le champs visuel étant mémorisées numériquement et le procédé d'examen présentant les étapes suivantes ;
a) Saisie des données de position pour une position de la tête du propositus dans le local ;
b) Sélection automatique d'une coupe de la zone du champ visuel associée à la position respective de la tête, et
c) Mise en représentation sur l'écran ou la surface de projection de la coupe sélectionnée de la zone du champs visuel avec les marques de fixation et les marques d'examen souhaitées, au choix avec le système de coordonnées servant de base.

2. Procédé suivant la revendication 1, **caractérisé en ce qu'**une sélection de la coupe des zones de champs visuel mémorisées du système de coordonnées de référence et des points de fixation, qui y sont déterminés, peut avoir lieu de manière dynamique, par mouvement de la tête, au moyen de circuits régulateurs optomoteurs du propositus, à savoir, par l'intermédiaire d'une biorégulation.

3. Procédé suivant la revendication 2, **caractérisé en ce que** la zone de champ visuel, représentée sur l'écran ou la surface de projection, continue à être déplacée par le mouvement de la tête, jusqu'à ce qu'un point de fixation apparaisse, auquel un examen doit avoir lieu.

4. Procédé suivant la revendication 3, **caractérisé par** le marquage du point de fixation, déjà pourvu d'un marquage au moyen d'un dispositif de marquage (5), pour l'examen par le propositus.

5. Procédé suivant une des revendications précédentes, **caractérisé par** le codage binaire des données de position saisies préalablement à la sélection de la coupe des coordonnées de champ visuel mémorisées.

6. Procédé suivant une des revendications précédentes, **caractérisé par** la sélection de la coupe des coordonnées de champs visuel mémorisées sur la base des données de position de la tête au moyen d'un tableau, qui détermine une association entre les données de position saisies et les valeurs des coordonnées de champs visuel mémorisées numériquement.

7. Procédé suivant une des revendications précédentes, **caractérisé par** la projection de la coupe sélectionnée de zones de champs visuels sur une surface de projection.

8. Disposition pour l'exécution du procédé suivant une des revendications précédentes, qui présente :
a) une mémoire pour la mémorisation numérique du système de coordonnées de référence nécessaire à la mesure de la déviation strabique ;
b) un dispositif de saisie (2) pour saisir les données de position pour une position de la tête du propositus dans le local ;
c) un dispositif de sélection pour la sélection automatique d'une coupe du système de coordonnées mémorisé en fonction des données de position saisies pour la position de la tête du propositus ;
d) un dispositif d'affichage (4), qui met en représentation la coupe sélectionnée du système de coordonnées mémorisé.
la mémoire et le dispositif de sélection étant prévus dans un ordinateur (3), auquel les données de position sont amenées par le dispositif de saisie (2).

9. Disposition suivant la revendication 8, **caractérisée en ce que** le dispositif d'affichage (4) est un écran ou un dispositif de projection.

10. Disposition suivant la revendication 8 ou 9, **caractérisée en ce qu'**est prévu, en outre, un dispositif de marquage (5) sur l'ordinateur (3), au moyen duquel le propositus marque un endroit d'examen déjà marqué dans le système de coordonnées.

11. Disposition suivant l'une des revendications 8 à 10, **caractérisée en ce que** le dispositif de saisie (2) saisit les ondes dirigées, émises par une source (9), disposée sur la tête du propositus avec un écart fixe par rapport aux yeux de celui-ci, et les transmet à l'ordinateur (3).

12. Disposition suivant la revendication 11, **caractérisée en ce que** les ondes dirigées sont des rayons lumineux, de préférence, des rayons laser.

13. Disposition suivant l'une des revendications 11 ou 12, **caractérisée en ce que** le dispositif de saisie (2) présente une caméra vidéo (8), qui saisit les données de position par balayage de points d'incidence (7) des ondes dirigées sur la surface d'incidence (7) conçue comme plateau de verre dépoli.

14. Disposition suivant l'une des revendications 8 à 11, **caractérisée en ce que** le dispositif de saisie (2) est un système à ultrasons ou un système à capteur d'inclinaison.
